(19) Europäisches Patentamt
European Patent Office
Office européen des brevets

(11) **EP 2 486 931 B1**

(12) **EUROPEAN PATENT SPECIFICATION**

(45) Date of publication and mention
of the grant of the patent:
**14.05.2014 Bulletin 2014/20**

(51) Int Cl.:
*A61K 35/74* *(2006.01)*     *A61K 31/716* *(2006.01)*
*A61K 45/00* *(2006.01)*     *A61P 31/16* *(2006.01)*

(21) Application number: **10822105.2**

(86) International application number:
**PCT/JP2010/067680**

(22) Date of filing: **07.10.2010**

(87) International publication number:
**WO 2011/043435 (14.04.2011 Gazette 2011/15)**

(54) **THERAPEUTIC AGENT FOR INFLUENZA VIRUS INFECTIOUS DISEASES**

THERAPEUTIKUM FÜR INFEKTIONSERKRANKUNGEN DURCH INFLUENZA-VIRUS

AGENT THÉRAPEUTIQUE POUR DES MALADIES INFECTIEUSES CAUSÉES PAR LE VIRUS DE LA GRIPPE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.10.2009   JP 2009234023**
**20.11.2009   JP 2009265049**

(43) Date of publication of application:
**15.08.2012   Bulletin 2012/33**

(73) Proprietor: **Aureo Co., Ltd.**
**Tokyo 108-0071 (JP)**

(72) Inventors:
• **MORIYA Naoyuki**
  **Kimitsu-shi**
  **Chiba 292-1149 (JP)**
• **MORIYA Yukiko**
  **Kimitsu-shi**
  **Chiba 292-1149 (JP)**
• **NIKAWA Yasuhiro**
  **Kimitsu-shi**
  **Chiba 292-1149 (JP)**

• **NISHINO Tamiko**
  **Kimitsu-shi**
  **Chiba 292-1149 (JP)**
• **KUSANO Kisato**
  **Kimitsu-shi**
  **Chiba 292-1149 (JP)**
• **MIYAZAKI Tadaaki**
  **Sapporo-shi**
  **Hokkaido 001-0020 (JP)**

(74) Representative: **Denison, Christopher Marcus et al**
**Mewburn Ellis LLP**
**33 Gutter Lane**
**London**
**EC2V 8AS (GB)**

(56) References cited:
**WO-A1-2008/053728     WO-A1-2008/112775**
**WO-A1-2009/005095     JP-A- 2002 204 687**
**JP-A- 2005 220 065     JP-A- 2006 075 076**
**JP-A- 2006 104 439     JP-A- 2008 013 543**
**JP-A- 2008 308 438**

**Description**

Technical Field

The present invention relates to a therapeutic agent for an influenza virus infectious disease.

Background Art

An influenza virus infectious disease causes symptoms such as sore throat, nasal discharge, high fever, headache, and arthralgia, and the symptoms may become serious in combination with bronchitis, pneumonia, or the like. In particular, the disease has a high mortality for elderly people or the like. In addition, group infection with the virus in schools and elderly facilities causes a social epidemic involving many people.

A conventional basic countermeasure for the influenza virus infectious disease is prevention by vaccination. However, antigenicity of the influenza virus is easily altered, and it has been difficult to completely control the epidemic only by vaccination. Further, it is difficult to produce a large amount of a vaccine in a short period of time, and there is a problem in that no countermeasure exists in a case of, for example, emergence of a novel influenza virus or an epidemic of an unexpected strain of the virus.

In recent years, to solve such problems, there have been used anti-influenza virus drugs such as oseltamivir (product name "Tamiflu," Chugai Pharmaceutical Co., Ltd.), amantadine (product name "Symmetrel," Novartis Pharma K.K.), and zanamivir (product name "Relenza," GlaxoSmithKline). However, the drugs have been found to have problems such as unavailability because their effects may be less effective if the drugs are not administered within 48 hours of the onset of the disease, side effects including ones having unclear causal relationships, and appearance of a resistant virus.

On the other hand, Patent Literature 1 below describes that a culture containing β-1,3-1,6-glucan, obtained by culturing a bacterium belonging to *Aureobasidium* sp., is used as an active ingredient for an immunopotentiator.

Citation List

Patent Literature

[PTL 1] JP 2005-220065 A

Summary of Invention

Technical Problem

A conventional antivirus composition utilizing an active ingredient derived from a natural product cannot be said to have sufficient effectiveness.

An object of the present invention is to provide a therapeutic agent for an influenza virus infectious disease, which utilizes an active ingredient derived from a natural product and has an excellent effect.

Solution to Problem

The inventors of the present invention have made extensive studies to achieve the object, and as a result, have completed the present invention. That is, the present invention is as follows.

[1] A therapeutic agent for an influenza virus infectious disease, including, as an active ingredient, a β-glucan-containing composition obtained from a culture of a microorganism belonging to *Aureobasidium* sp. , the therapeutic agent being used for preventing an influenza virus infectious disease from becoming serious and for promoting healing of the influenza virus infectious disease.

[2] A therapeutic agent for an influenza virus infectious disease according to the above-mentioned item [1], in which the microorganism belonging to *Aureobasidium* sp. is *Aureobasidium pullulans* M-1 having an accession number of FERM BP-08615 or *Aureobasidium pullulans* M-2 having an accession number of FERM BP-10014.

[3] A therapeutic agent for an influenza virus infectious disease according to the above-mentioned item [1] or [2], further including, as another active ingredient, bacterial cells of a lactic acid bacterium.

[4] A therapeutic agent for an influenza virus infectious disease according to the above-mentioned item [3], in which the bacterial cells of a lactic acid bacterium are dead bacterial cells of *Enterococcus faecalis.*

[5] A therapeutic agent for an influenza virus infectious disease according to any one of the above-mentioned items [1] to [4], which is administered in combination with at least one kind selected from the group consisting of an influenza neuraminidase inhibitor, an antiviral drug, an antibiotic, and a steroid drug.

Advantageous Effects of Invention

According to the therapeutic agent for an influenza virus infectious disease of the present invention, it is possible to prevent an influenza virus infectious disease from becoming serious and to promote the healing of the disease. The active ingredient in the agent is a β-glucan-containing composition obtained from a culture of a microorganism belonging to the *Aureobasidium* sp. Therefore, its excellent effect can be provided safely without a risk of side effects even if the agent is administered for a long period of time and even in an easy administration form. In addition, administration of the agent in combination with bacterial cells of a lactic acid bacterium can further enhance the effect. Further, administration of the agent in combination with at least one kind selected from the group consisting of an influenza neuraminidase inhibitor, an antiviral drug, an antibiotic, and a steroid drug can further enhance the effect.

Brief Description of Drawings

[FIG. 1] A graph showingshowing results of variations in body weights of mice when a culture solution of *Aureobasidium pullulans* M-1 is administered to mice infected with an influenza virus (Test group 1) (Test Example 1).

[FIG. 2] A graph showing results of variations in body weights of mice when an anti-influenza drug oseltamivir (product name "Tamiflu, "Chugai Pharmaceutical Co., Ltd.) is administered to mice infected with the influenza virus (Test group 2) (Test Example 1).

[FIG. 3] A graph showing results of variations in body weights of mice when phosphate buffered saline (PBS) is administered to mice infected with the influenza virus (Control group 1) (Test Example 1).

[FIG. 4] A graph showing results of variations in body weights of mice when the culture solution of *Aureobasidium pullulans* M-1 is administered to mice infected with the influenza virus (Test group 3) (Test Example 2).

[FIG. 5] A graph showing results of variations in body weights of mice when a culture solution of *Aureobasidium pullulans* M-2 is administered to mice infected with the influenza virus (Test group 4) (Test Example 2).

[FIG. 6] A graph showing results of variations in body weights of mice when the culture solution of *Aureobasidium pullulans* M-1 and heat-sterilized bacterial cells of *Enterococcus faecalis* are administered in combination to mice infected with the influenza virus (Test group 5) (Test Example 2).

[FIG. 7] A graph showing results of variations in body weights of mice when the culture solution of *Aureobasidium pullulans* M-1 and an anti-influenza drug oseltamivir (product name "Tamiflu," Chugai Pharmaceutical Co., Ltd.) are administered in combination to mice infected with the influenza virus (Test group 6) (Test Example 2).

[FIG. 8] A graph showing results of variations in body weights of mice when the anti-influenza drug oseltamivir (product name "Tamiflu, "Chugai Pharmaceutical Co., Ltd.) is administered to mice infected with the influenza virus (Test group 7) (Test Example 2).

[FIG. 9] A graph showing results of variations in body weights of mice when phosphate buffered saline (PBS) is administered to mice infected with the influenza virus (Control group 2) (Test Example 2).

[FIG. 10] A graph showing transition of survival rates of mice of the respective test groups during a test period of Test Example 2.

[FIG. 11] A graph showing results of variations in body weights of mice when the culture solution of *Aureobasidium pullulans* M-2 and heat-sterilized bacterial cells of *Enterococcus faecalis* are administered in combination to mice infected with the influenza virus (Test group 8) (Test Example 3).

[FIG. 12] A graph showing results of variations in body weights of mice when the culture solution of *Aureobasidium pullulans* M-2 and heat-sterilized bacterial cells of *Enterococcus faecalis* are administered in combination to mice infected with the influenza virus (Test group 9) (Test Example 3).

[FIG. 13] A graph showing results of variations in body weights of mice when the culture solution of *Aureobasidium pullulans* M-2 and heat-sterilized bacterial cells of *Enterococcus faecalis* are administered in combination to mice infected with the influenza virus (Test group 10) (Test Example 3).

[FIG. 14] A graph showing results of variations in body weights of mice when the culture solution of *Aureobasidium pullulans* M-2 and heat-sterilized bacterial cells of *Enterococcus faecalis* are administered in combination to mice infected with the influenza virus (Test group 11) (Test Example 3).

[FIG. 15] A graph showing results of variations in body weights of mice when heat-sterilized bacterial cells of *Enterococcus faecalis* are administered to mice infected with an influenza virus (Test group 12) (Test Example 3).

[FIG. 16] A graph showing results of variations in body weights of mice when phosphate buffered saline (PBS) is administered to mice infected with an influenza virus (Control group 3) (Test Example 3).

[FIG. 17] A graph showing transition of survival rates of mice of the respective test groups during a test period of Test Example 3.

[FIG. 18] A graph showing virus titers in lungs of mice of the respective test groups of Test Example 4.

[FIG. 19] A graph showing ratios of NK cells relative to viable cells in lungs of mice of the respective test groups of Test Example 5.

[FIG. 20] A graph showing ratios of activated NK cells relative to NK cells in lungs of mice of the respective test groups of Test Example 5.

[FIG. 21] A graph showing ratios of T cells relative to viable cells in lungs of mice of the respective test groups of Test Example 5.

[FIG. 22] A graph showing ratios of activated T cells relative to T cells in lungs of mice of the respective test groups of Test Example 5.

Description of Embodiments

A therapeutic agent for an influenza virus infectious disease of the present invention contains, as an active ingredient, a β-glucan-containing composition obtained from a culture of a microorganism belonging to *Aureobasidium* sp. (hereinafter, referred to as "β-glucan-containing composition derived from *Aureobasidium*"). The β-glucan-containing composition derived from *Aureobasidium* includes not only a culture itself obtained by culturing a microorganism that belongs to the *Aureobasidium* sp. and has a β-glucan producing ability (hereinafter, referred to as "*Aureobasidium* microorganism"), a culture solution obtained by separating and removing microorganism cells by centrifugation or the like, a concentrated solution of the culture solution, a diluted solution of the culture solution, solid matter obtained by removing water from the culture solution, or the like but also a product obtained by, for example, demineralizing any of the foregoing to increase the content of β-glucan or a purified β-glucan product obtained from any of the foregoing.

The β-glucan-containing composition derived from *Aureobasidium* to be used in the present invention contains β-glucan produced by culturing the *Aureobasidium* microorganism in an amount of preferably 50 to 3,000 mg, more preferably 100 to 2,000 mg in terms of a content relative to 100 g (mass) of the culture.

It should be noted that the content of β-glucan can be determined in accordance with, for example, the following method. Specifically, a culture solution is subjected to enzyme treatment with amylase, amyloglucosidase, protease, or the like, and proteins and α-glucan such as pullulan are removed, followed by ethanol precipitation. Further, the resultant is filtered using a glass filter, to thereby yield a high-molecular-weight sample. At this time, the sample is sufficiently washed with 80% ethanol in order to remove low-molecular-weight substances including monosaccharides. The washed high-molecular-weight sample is further washed with acetone, and sulfuric acid is added thereto for hydrolysis. After the hydrolysis, neutralization is carried out, and the filtrate is collected. Quantification of glucose is carried out by a glucose oxidase method, and a value calculated based on the following mathematical expression 1 is defined as the β-glucan amount.

```
Mathematical Expression 1: β-glucan (g/100 g)=glucose (g/100 g)×0.9
```

Moreover, the content of β-glucan may also be determined as an amount of a so-called sugar chain-containing high-molecular-weight substance (polysaccharide). In such case, a culture solution is subjected to enzyme treatment with amylase, amyloglucosidase, protease, or the like, and proteins and α-glucan such as pullulan are removed, followed by ethanol precipitation. Further, the resultant is filtered using a glass filter, to thereby yield a high-molecular-weight sample. At this time, the sample is sufficiently washed with 80% ethanol in order to remove low-molecular-weight substances including monosaccharides. The washed high-molecular-weight sample is further washed with acetone, and the weight of the resultant sample is determined as an amount of a sugar chain-containing high-molecular-weight substance (polysaccharide).

It should be noted that the β-glucan quantified as described above is quantified as a substance containing a functional group such as a sulfate group or a phosphate group. Therefore, in the case where the β-glucan is quantified as a broad-sense sugar chain-containing high-molecular-weight substance (polysaccharide) as described above, the content of the β-glucan produced by culturing the *Aureobasidium* microorganism is preferably 70 to 5, 000 mg, more preferably 140 to 3,000 mg in terms of a content relative to 100 g (mass) of the culture.

The *Aureobasidium* microorganism to be used in the present invention has only to be a microorganism belonging to *Aureobasidium* sp. and having a β-glucan producing ability. For example, *Aureobasidium pullulans* M-1 (the National Institute of Advanced Industrial Science and Technology International Patent Organism Depositary, Chuo Dai-6, 1-1 Higashi 1-Chome, Tsukuba-shi, Ibaraki-ken, Japan, accession number: FERMBP-08615, international deposit on February 10, 2004) or, *Aureobasidium pullulans* M-2 (the National Institute of Advanced Industrial Science and Technology International Patent Organism Depositary, Chuo Dai-6, 1-1 Higashi 1-Chome, Tsukuba-shi, Ibaraki-ken, Japan, accession number: FERM BP-10014, international deposit on April 22, 2004) is suitably employed. It shoulddbe noted that, β-glucan produced by these strains of microorganisms has been shown to be β-1, 3-1, 6-glucan, which has a structure in which glucose is branched with a β-1,6 bond from a main chain in which glucose molecules are linked by a β-1,3 bond from a structural analysis by NMR measurement (13C NMR :Varian, UNITY INOVA500, 1H NMR : Varian, UNITY INOVA600).

The *Aureobasidium* microorganism can be cultured in accordance with a known method (see, for example, JP 57-149301 A). That is, the microorganism is inoculated in a medium (pH 5.2 to 6. 0) supplemented with 0. 5 to 5. 0% by mass of a carbon source (sucrose), 0.1 to 5.0% by mass of an N source, and other trace substances (for example, vitamins or minerals). The microorganism is cultured at a temperature of 20 to 30° C for 2 to 14 days with aeration, preferably with aeration and stirring. The viscosity of the culture solution becomes higher as β-glucan is produced, resulting in a gel-like composition having an increased viscosity. The culture solution thus obtained generally contains 0.6 to 10% by mass of solid matter, and the solidmatter contains 5 to 80% by mass of β-glucan. Moreover, the solid matter contains not only β-glucan but also other useful ingredients such as phosphorus, potassium, magnesium, and vitamin C, which are ingredients for aiding an action of the glucan. Hence, a physiologically active effect of β-glucan can be exerted efficiently.

In the present invention, the culture thus obtained itself may be used after sterilization by heating without pressure, or after sterilization by heating under pressure. Alternatively, the culture solution may be sterilized and used after the separation and removal of microorganism cells by centrifugation or the like. Moreover, the culture may be used after concentration or after concentration and drying, if necessary. Further, only β-glucan may be extracted and used. It should be noted that the culture of a microorganism belonging to the *Aureobasidium* sp. is used as a food additive such as a thickener, and has high safety.

With regard to its application method, when the composition is prepared for oral administration, for example, it is possible to exert its physiologically active effect in the body through oral ingestion thereof.

The amount of the composition to be administered can be appropriately determined depending on, for example, the purpose of treatment or prevention, the degree of the symptom, the age of a patient, the administration method, the frequency of administration, and the administration period. For example, in the case of oral ingestion, a general effective dose is about 0.06 to 60 mg/kg (body weight) per day in terms of the amount of β-glucan. The therapeutic agent for an influenza virus infectious disease of the present invention is effective for prevention and treatment of complications caused after infection with an influenza virus. Examples of the complications include otitis media, sinusitis, bronchitis, exacerbation of chronic bronchitis, pneumonia, encephalopathy, and renal failure. In particular, the agent is effective for prevention of a disease caused by a so-called cytokine storm or a tissue-destroying disease (disease caused by TH1 enhancement).

The therapeutic agent for an influenza virus infectious disease of the present invention may further contain bacterial cells of a lactic acid bacterium as an active ingredient other than the culture of *Aureobasidium.* Such agent can be expected to have a synergistic action with a physiologically active effect of the lactic acid bacterium.

A lactic acid bacterium or the like applicable to food and having no problem in safety is employed as the lactic acid bacterium. Specific examples thereof may include *Enterococcus faecalis, Enterococcus faecium, Lactobacillus acidophilus, Lactobacillus casei, Lactobacillus brevis, Streptococcus cremoris, Streptococcus lactis, Streptococcus thermophilus, Bifidobacterium Longum, Bifidobacterium breve,* and *Bifidobacterium bifidum.* The lactic acid bacteria may be used alone or in combination of two or more kinds thereof.

It should be noted that *Enterococcus faecalis* and *Enterococcus faecium* are lactic acid bacteria used in lactic acid bacterium preparations or the like, *Lactobacillus casei* and *Lactobacillus acidophilus* are lactic acid bacteria used in cheese, fermented milk, yogurt, lactic acid bacterium drinks, or the like, *Streptococcus cremoris, Streptococcus lactis,* and *Streptococcus thermophilus* are lactic acid bacteria used in cheese, yogurt, or the like, and *Bifidobacterium Longum, Bifidobacterium breve,* and *Bifidobacterium bifidum* are lactic acid bacteria used in fermented milk or the like. Thus, all of these lactic acid bacteria are easily obtainable for those skilled in the art.

In addition, as the lactic acid bacterium, not only viable cells but also heat-sterilized dead bacterial cells may be used. In the case where the dead bacterial cells are used, preservation stability is enhanced, and safety is enhanced when the cells are used as raw materials for drugs and functional foods. As the dead bacterial cells of the lactic acid bacterium, dead bacterial cells of *Enterococcus faecalis* and *Lactobacillus brevis* subspecies *coagulans* are commercially available and preferably used.

The lactic acid bacterium may be cultured in accordance with a conventional method. In addition, dead bacterial cells may be obtained by collecting bacterial cells from a culture of the lactic acid bacterium by means of a method such as filtration or centrifugation, washing the cells with water, and then suspending the cells in water or the like, followed by heat sterilization at 80 to 115°C for 30 minutes to 3 seconds. The heat-sterilized lactic acid bacterium may be used after concentration or drying, if necessary.

The amount of the lactic acid bacterium to be administered can be appropriately determined depending on, for example, the purpose of treatment or prevention, the degree of the symptom, the age of a patient, the administration method, the frequency of administration, and the administration period. For example, in the case of oral ingestion, a general effective dose is about $1 \times 10^9$ to $4 \times 10^{11}$ cells/kg (body weight), more preferably $2 \times 10^9$ to $1 \times 10^{11}$ cells/kg (body weight) per day in terms of the amount of dry dead bacterial cells.

In the present invention, of the above-mentioned lactic acid bacteria, dead bacterial cells of *Enterococcus faecalis* are particularly preferably used. The dead bacterial cells of *Enterococcus faecalis* are known to have a strong immunopotentiating activity, and when the cells are used in combination with the culture of *Aureobasidium,* a synergistic effect of them may be expected in anti-influenza virus effect as well.

In the present invention, as a form of administration of the therapeutic agent, the agent maybe administered in combination with at least one kind selected from the group consisting of an influenza neuraminidase inhibitor, an antiviral drug, an antibiotic, and a steroid drug. According to this form, a synergistic effect with the anti-influenza virus effect of such drug may be expected. Preferred examples of the influenza neuraminidase inhibitor may include oseltamivir and zanamivir. Preferred examples of the antiviral agent may include amantadine. Preferred examples of the antibiotic may include clarithromycin, cefaclor, cefdinir, cefpodoxime proxetil, cefotiam hydrochloride, cefcapene pivoxil hydrochloride, cefditoren pivoxil, cefmetazole sodium, cefotiam hydrochloride, cefozopran hydrochloride, pivmecillinam hydrochloride, sultamicillin tosilate, fosfomycin calcium, isepamicin sulfate, gentamicin sulfate, and kanamycin monosulfate. Preferred examples of the steroid drug may include methylprednisolone.

In the present invention, in the case where, as a form of administration of the therapeutic agent, the agent is administered in combination with at least one kind selected from the group consisting of an influenza neuraminidase inhibitor, an antiviral drug, an antibiotic, and a steroid drug, the culture of *Aureobasidium* may be administered while the antiviral drugs are administered in suitable modes for the respective antiviral drugs. In this case, the culture of *Aureobasidium* shows no outstanding side effects, and hence the antiviral drugs may be administered for a relatively short period of time while the culture is administered continuously for a relatively long period of time.

Examples

Hereinafter, the present invention is specifically described by way of examples. However, these examples are not intended to limit the scope of the present invention.

(Production Example 1)

A culture solution of *Aureobasidium pullulans* M-1 (FERM BP-08615) was prepared as follows.

An appropriate amount of a pre-culture was inoculated in a liquidmedium (pH 5.3) containing 1% of sucrose, 0.1% of ascorbic acid, and 0.1% of rice bran, and cultured with aeration and stirring at 25°C for 72 to 96 hours (varying depending on production batches) . After completion of the culture, the culture solution was sterilized at 121°C for 15 minutes. The resultant sterilized culture solution contained about 1.2% by mass of solid matter, and the content of β-glucan in 100 g of the solid matter was 16.7 g. In addition, the content of β-glucan was 0.2 g/100 g in terms of a content in 100 g (mass) of the culture solution itself. The culture solution was used in the following test.

(Test Example 1)

Test solutions were prepared for the test groups and control group shown in Table 1 below, respectively, and effects of oral administration of the solutions on mice infected with an influenza virus were examined. It should be noted that "Tamiflu dry syrup 3%" (product name, Chugai Pharmaceutical Co., Ltd.) was used as an anti-influenza drug oseltamivir.

[Table 1]

|  | Test solution | Form | Concentration of β-glucan |
|---|---|---|---|
| Test group 1 | Culture solution of *Aureobasidium* (M-1) | Culture solution itself | 0.2 g/100 g |
| Test group 2 | 0.5 mg oseltamivir/1mL | PBS suspension | - |
| Control group 1 | Phosphate buffered saline (PBS) | - | - |

As experimental animals, mice (C57BL/6NJcl, male, 6 weeks old) were used, and as the influenza virus, H1N1 subtype of influenza virus A/PR/8 was used. Infection of the mice with the influenza virus was carried out as follows. That is, the mice were placed in a container filled with isoflurane, and taken out after confirming that their breathing became slow. A diluted virus solution was prepared by serial dilution with PBS so that the solution had a virus titer of $2\times10^4$ pfu/mL, and injected from both nasal cavities alternately using a micropipette in an amount of 50 μL (1,000 pfu/mouse). After confirming that the mice recovered from anesthesia, the mice were returned to breeding cages.

The test solutions were administered in the forms and by the methods shown in Table 2 below. It should be noted that the culture solution of *Aureobasidium* (Test group 1) was orally administered every day during the test period by a sonde method once a day in an amount of 200 μL. The anti-influenza drug oseltamivir (Test group 2) was orally administered in accordance with a method which was considered to be the most effective as its administration method (see "DIRK B. MENDEL et al., ANTIMICROBIAL AGENTS AND CHEMOTHERAPY, Mar. 1998, p.640-646") for five days after infection with the virus by the sonde method twice a day (morning and evening, in an amount of 200 μL per dose). The body weights of the mice after infection with the virus were measured every day, and transition of the body weights and survival rates were determined.

[Table 2]

| | Administration form and method | | | | | |
|---|---|---|---|---|---|---|
| | For two weeks before the day of virus infection (per day) | For five days including the day of virus infection (per day) | From day 6 of virus infection to the second week (per day) | Administration period | Dose per day | Number of mice |
| Test group 1 | Culture solution 200 μl | Culture solution 200 μl | Culture solution 200 μl | β-glucan four weeks | β-glucan 0.4 mg | 3 |
| Test group 2 | PBS 200 μl | Oseltamivir 200 μl (twice in morning and evening, 400 μl in total) | PBS 200 μl | Oseltamivir five days | Oseltamivir 0.2 mg | 3 |
| Control group 1 | PBS 200 μl | PBS 200 μl | PBS 200 μl | - | - | 3 |

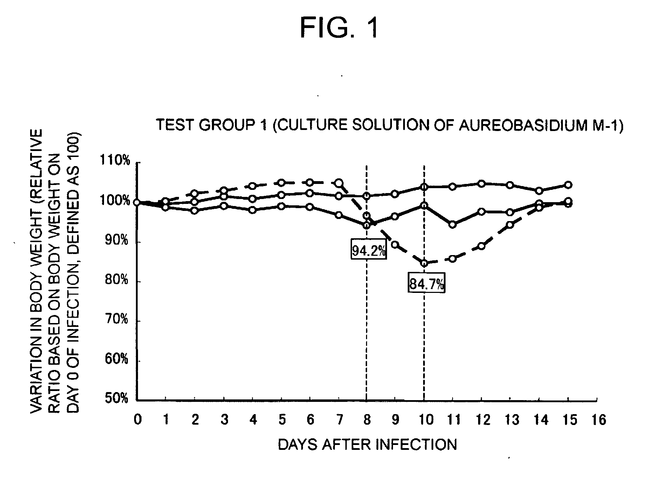

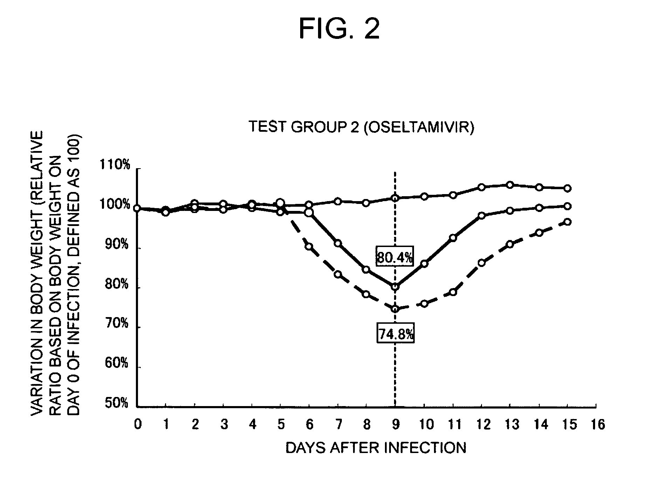

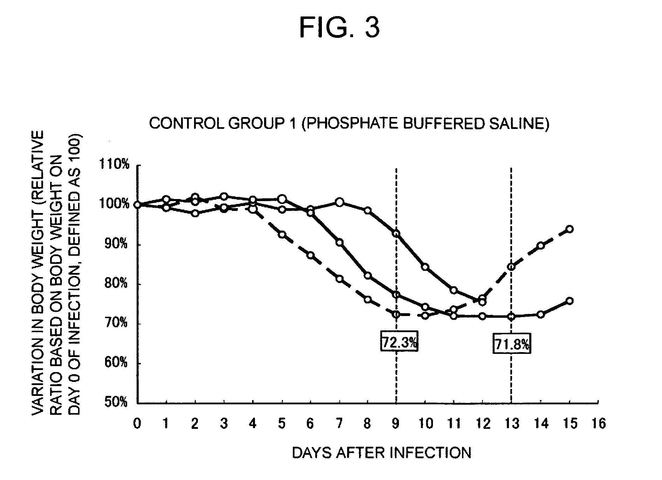

FIG. 1 illustrates the results of Test group 1, FIG. 2 illustrates the results of Test group 2, and FIG. 3 illustrates the results of Control group 1.

As illustrated in FIG. 1, the survival rate of Test group 1 was 100%. After infection, the body weight of one mouse increased by 5% at the end of the test, i.e., on day 14 of infection as compared to the first day of infection without ever falling below the body weight at the time of infection. In the case of another mouse, the body weight started to decrease from about day 7 of infection and decreased by up to about 15% three days after the day, i.e., on day 10 of infection. However, after that, the body weight recovered, and the test was completed at the same body weight as that on the first day of infection. In the case of the other mouse, the body weight started to decrease from day 5 or day 6 of infection and decreased by up to about 6% two days after the day, i.e., on day 8 of infection. After that, the body weight recovered, and the test was completed at the same body weight as that on the first day of infection.

As illustrated in FIG. 2, the survival rate of Test group 2 was 100%. After infection, the body weight of one mouse increased by 5% at the end of the test, i.e., on day 14 of infection as compared to the first day of infection without ever falling below the body weight at the time of infection. In the case of another mouse, the body weight started to decrease from about six days after infection and decreased by up to about 20% three days after the day, i.e., on day 9 of infection. However, after that, the body weight recovered, and the test was completed at the same body weight as that on the first day of infection. In the case of the other mouse, the body weight started to decrease from day 5 of infection and decreased by up to about 25% four days after the day, i.e., on day 9 of infection. However, after that, the body weight recovered, and the test was completed when the body weight decreased by 3% as compared to the first day of infection.

As illustrated in FIG. 3, the survival rate of Control group 1 was 66.6% (one of three mice died). After infection, the body weight of one mouse started to decrease significantly from day 7, and the mouse died on day 12 of infection without recovering the body weight. In the case of another mouse, the bodyweight started to decrease from day 4 of infection, and decreased by up to about 28% five days after the day, i.e., on day 9 of infection. However, after that, the body weight recovered, and the test was completed when the body weight decreased by 6% as compared to the first day of infection. In the case of the other mouse, the body weight started to decrease from day 5 of infection and decreased by up to about 28% during a period from day 11 of infection to day 14 of infection. However, the test was completed when the body weight recovered slightly on day 14 of infection.

The above-mentioned results are summarized as follows: administration of the culture solution of *Aureobasidium* suppressed a symptom caused by infection with the influenza virus, i.e., loss of the body weights of the mice and promoted recovery from the loss of the body weights. In addition, the effect of the culture solution was found to be equal to or larger than that of the anti-influenza virus drug oseltamivir (product name "Tamiflu," Chugai Pharmaceutical Co., Ltd.). Therefore, it was found that the β-glucan-containing composition obtained from the culture of the microorganism belonging to the *Aureobasidium* is effective for treatment of an influenza virus infectious disease, in particular, for preventing the disease from becoming serious and promoting the healing of the disease after infection with the virus.

Although the mechanism of the β-glucan-containing composition derived from *Aureobasidium* to exert the effect is unknown, it was considered that the composition enhances not only actions caused by immunostimulation such as macrophage phagocytic capacity activation but also immune balance functions inherent in the body to maintain a balance between, for example, Th1 cell immunity and Th2 cell immunity, resulting in suppressing unnecessary attack on self-tissues by excessive immune responses.

(Production Example 2)

A culture solution of *Aureobasidium pullulans* M-2 (FERM BP-10014) was prepared in the same manner as in Production Example 1. The culture solution contained about 1.2% by mass of solidmatter, and the content of β-glucan in 100 g of the solid matter was 54.2 g. In addition, the content of β-glucan was 0.65 g/100 g in terms of a content in 100 g (mass) of the culture solution itself. The culture solution was used in the following test.

(Test Example 2)

Test solutions were prepared for the test groups and control group as shown in Table 3 below, respectively, and effects of oral administration of the solutions on mice infected with an influenza virus were examined. It should be noted that the phrase "dead bacterial cells of EF lactic acid bacterium" in the table means a commercially available heat-sterilized bacterial cell powder of *Enterococcus faecalis.*

[Table 3]

| | Test solution | Form | Concentration of β-glucan |
|---|---|---|---|
| Test group 3 | Culture solution of *Aureobasidium* (M-1) | Culture solution itself | 0.2 g/100 g |
| Test group 4 | Culture solution of *Aureobasidium* (M-2) | Culture solution diluted with PBS | 0.2 g/100 g |
| Test group 5 | Culture solution of *Aureobasidium* (M-1) + dead bacterial cells of EF lactic acid bacterium $6.7 \times 10^9$ cells | Suspension of dead bacterial cells of EF lactic acid bacterium in culture solution | 0.2 g/100 g |
| Test group 6 | Culture solution of *Aureobasidium* (M-1) + 0.5 mg oseltamivir/1 mL | Suspension of oseltamivir in culture solution | 0.2 g/100 g |
| Test group 7 | 0.5 mg/1 mL oseltamivir | PBS suspension | - |
| Control group 2 | Phosphate buffered saline (PBS) | - | - |

Infection of mice used as experimental animals with the influenza virus was carried out so that the virus titer was twice as large as that in Test Example 1. That is, the infection was carried out in the same manner as in Test Example 1 except that a diluted virus solution having a virus titer of $4 \times 10^4$ pfu/mL was injected from both nasal cavities alternately using a micropipette in an amount of 50 μL (2,000 pfu/mouse).

The test solutions were administered in the forms and by the methods shown in Table 4 below. That is, in the case of each of Test groups 3 to 5 and Control group 2, the test solution was orally administered every day during the test period by the sonde method once a day in an amount of 200 μL. In the case of Test group 6, to which the culture solution of *Aureobasidium* was administered in combination with oseltamivir, in order to administer oseltamivir effectively, in the same manner as in Test group 2 in Test Example 1, a suspension of oseltamivir in the culture solution of *Aureobasidium* was orally administered twice a day (morning and evening, in an amount of 200 μL per dose) for five days after infection with the virus, and during the remaining period, only the culture solution of *Aureobasidium* was orally administered once a day in an amount of 200 μL. Further, in the case of Test group 7, to which only oseltamivir was administered, in order to carry out comparison with the maximum effect of administration of only oseltamivir, oseltamivir, was orally administered every day (morning and evening, in an amount of 200 μL per dose) continuously for 17 days after infection with the virus.

The body weights of the mice after infection with the virus were measured every day, and transition of the body weights and survival rates were determined.

[Table 4]

| | | Administration form and method | | | | | |
|---|---|---|---|---|---|---|---|
| | | For two weeks before the day of virus infection (per day) | For five days including the day of virus infection (per day) | From day 6 to day 17 of virus infection (per day) | Administration period | Dose per day | Number of mice |
| Test group 3 | | Culture solution 200 μl | Culture solution 200 μl | Culture solution 200 μl | β-glucan 31 weeks | β-glucan 0.4 mg | 5 |
| Test group 4 | | Culture solution 200 μl | Culture solution 200 μl | Culture solution 200 μl | β-glucan 31 weeks | β-glucan 0.4 mg | 5 |
| Test group 5 | | Culture solution + lactic acid bacterium 200 μl | Culture solution 200 μl + lactic acid bacterium | Culture solution 200 μl + lactic acid bacterium | β-glucan 31 weeks Lactic acid bacterium 31 days | β-glucan 0.4 mg Lactic acid bacterium $6.7 \times 10^9$ cells | 4 |
| Test group 6 | | Culture solution 200 μl | Culture solution 200 μl + oseltamivir (twice in morning and evening, 400 μl in total) | Culture solution 200 μl | β-glucan 31 weeks Oseltamivir 5 days | β-glucan 0.4 mg Oseltamivir 0.2 mg | 4 |
| Test group 7 | | PBS 200 μl | Oseltamivir 200 μl (twice in morning and evening, 400 μl in total) | Oseltamivir 200 μl (twice in morning and evening, 400 μl in total) | Oseltamivir 17 days | Oseltamivir 0.2 mg | 4 |
| Control group 2 | | PBS 200 μl | PBS 200 μl | PBS 200 μl | - | - | 4 |

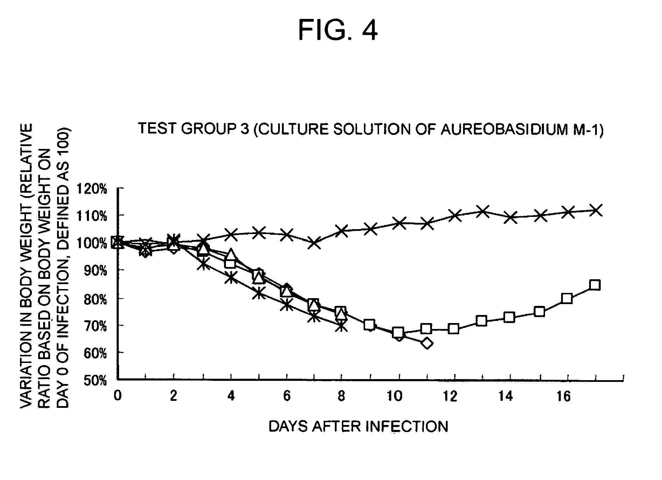

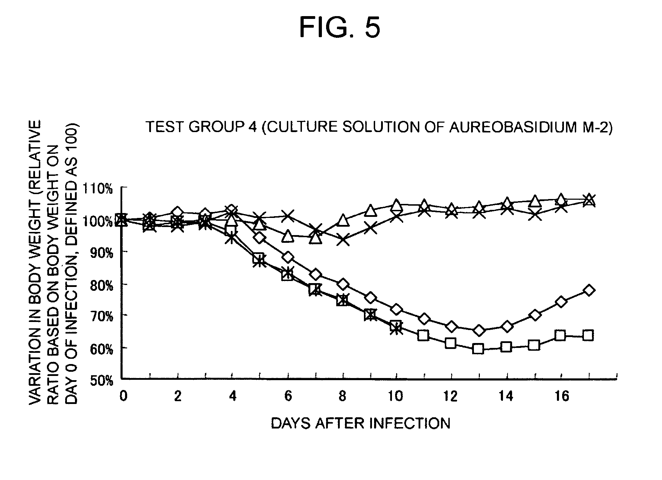

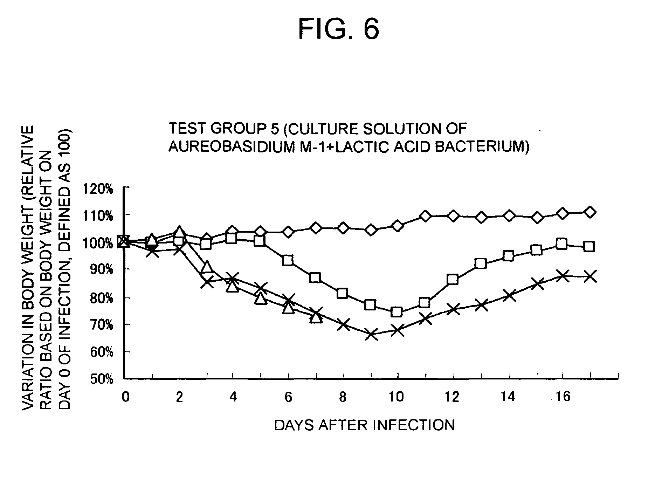

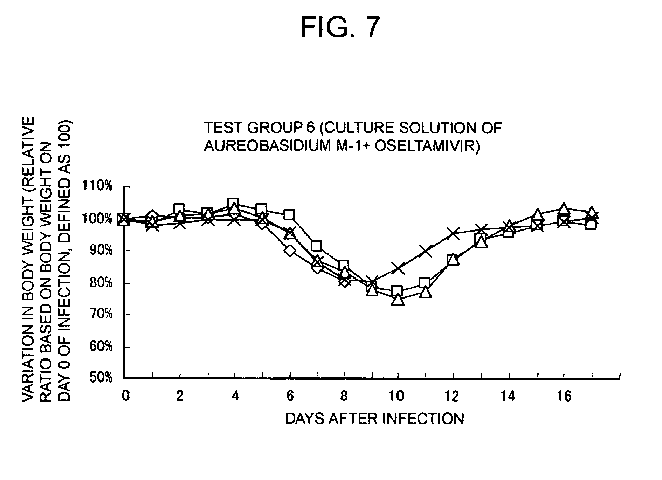

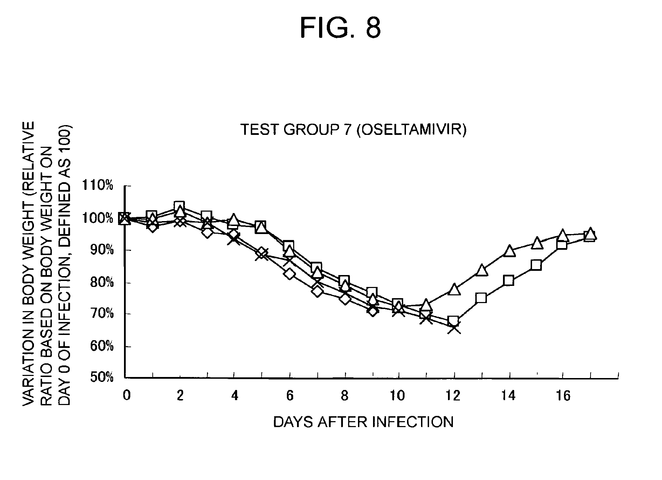

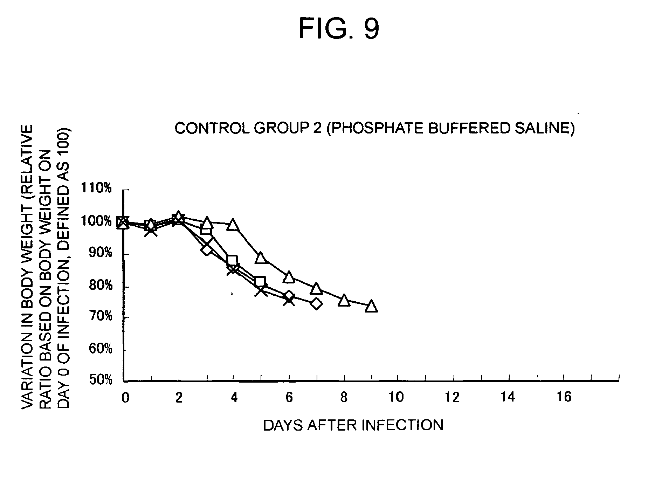

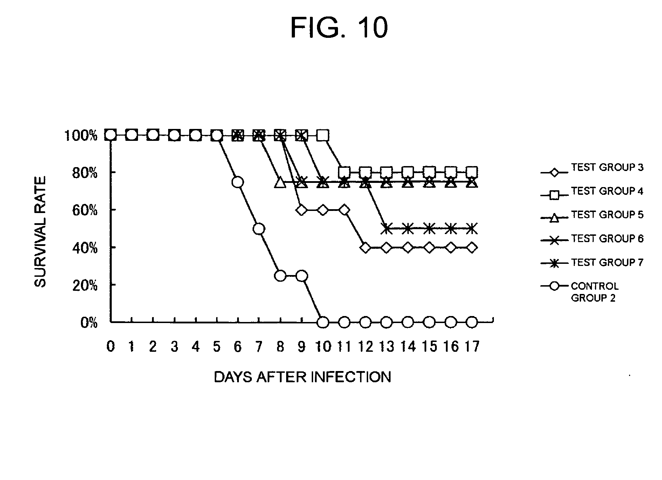

FIG. 4 illustrates the results of Test group 3, FIG. 5 illustrates the results of Test group 4, FIG. 6 illustrates the results of Test group 5, FIG. 7 illustrates the results of Test group 6, FIG. 8 illustrates the results of Test group 7, and FIG. 9 illustrates the results of Control group 2. Further, FIG. 10 illustrates transition of survival rates of mice of the respective groups.

In Test Example 2, as a result of infection of the mice carried out so that the virus titer was twice as large as that in Test Example 1, in the case of Control group 2, to which PBS was administered (FIG. 9), all the four mice died until day 10 of infection (FIG. 10).

Moreover, in the cases of Test group 3 (FIG. 4) and Test group 4 (FIG. 5), to each of which the culture solution of *Aureobasidium* was administered, four of ten mice in total (40%) of Test groups 3 and 4 died during the test period (FIG. 10). Further, the body weights of four of five mice in Test group 3 (FIG. 4) and three of five mice in Test group 4 (FIG. 5) decreased by up to about 41%. The mice whose body weights decreased died or completed the test while hardly recovering the body weights.

Further, in the case of Test group 7, to which oseltamivir was administered (FIG. 8), the body weights of four of four mice decreased by up to about 34%, and two of the four mice (50%) died during the test period (FIG. 10).

On the other hand, as illustrated in FIG. 7, in the case of Test group 6, to which the culture solution of *Aureobasidium* was administered in combination with oseltamivir, the body weights of all the four mice decreased during a period from day 6 to day 9 of virus infection. However, their body weights decreased by up to about 25% except for dead one of the four mice (25%) and then recovered to normal levels. In addition, in the case of Test group 5, to which the culture solution of *Aureobasidium* was administered in combination with the heat-sterilized bacterial cell powder of the lactic acid bacterium, the same tendency was observed. That is, only one of the four mice (25%) dead during the test period, and the body weights of three of the four mice decreased during a period from day 3 to day 10 of virus infection. After that, however, the body weights of the mice excluding the dead one mouse were significantly recovered to near normal levels.

The above-mentioned results show that the β-glucan-containing composition derived from *Aureobasidium* is effective for treating an influenza virus infectious disease, in particular, for preventing the disease from becoming serious and promoting the healing of the disease after virus infection, and the effect can be more enhanced by using bacterial cells of a lactic acid bacterium or oseltamivir in combination.

(Test Example 3)

An effect of administration of the culture of *Aureobasidium* in combination with bacterial cells of a lactic acid bacterium was further examined. Specifically, test solutions were prepared for the test groups and control group shown in Table 5 below, respectively, and orally administered in the same manner as in Test Example 2, and transition of body weights and survival rates of mice infected with the influenza virus were examined. It should be noted that the phrase "dead bacterial cells of EF lactic acid bacterium" in the table means a commercially available heat-sterilized bacterial cell powder of *Enterococcus faecalis.*

[Table 5]

|  | | Test solution | Form | Concentration of β-glucan |
|---|---|---|---|---|
| Test group 8 | | Culture solution of *Aureobasidium* (M-2) + dead bacterial cells of EF lactic acid bacterium $6.7\times10^9$ cells | Suspension of dead bacterial cells of EF lactic acid bacterium in culture solution | 0.2 g/100 g |
| Test group 9 | | Culture solution of *Aureobasidium* (M-2) + dead bacterial cells of EF lactic acid bacterium $10.1\times10^9$ cells | Suspension of dead bacterial cells of EF lactic acid bacterium in culture solution | 0.2 g/100 g |
| Test group 10 | | Culture solution of *Aureobasidium* (M-2) + dead bacterial cells of EF lactic acid bacterium $3.4\times10^9$ cells | Suspension of dead bacterial cells of EF lactic acid bacterium in culture solution | 0.2 g/100 g |
| Test group 11 | | Culture solution of *Aureobasidium* (M-2) + dead bacterial cells of EF lactic acid bacterium $6.7\times10^9$ cells | Suspension of dead bacterial cells of EF lactic acid bacterium in culture solution | 0.1 g/100 g |
| Test group 12 | | Dead bacterial cells of EF lactic acid bacterium $6.7\times10^9$ cells | PBS suspension | - |
| Control group 3 | | Phosphate buffered saline (PBS) | - | - |

The test solutions were administered in the forms and by the methods shown in Table 6 below. That is, in the case of Test group 8, the dead bacterial cells of the lactic acidbacterium ($6.7\times10^9$ cells) were suspended in 200 μL of the culture solution of *Aureobasidium pullulans* M-2 (FERM BP-10014) prepared so as to have a β-glucan concentration of 0.2 g/100 g, and the suspension was orally administered every day during the test period by the sonde method once a day. In the case of Test group 9, the test was carried out in the same manner as in Test group 8 except that the deadbacterial cells of the lactic acid bacterium ($10.1\times10^9$ cells) were suspended. In the case of Test group 10, the test was carried out in the same manner as in Test group 8 except that the dead bacterial cells of the lactic acid bacterium ($3.4\times10^9$ cells) were suspended. In the case of Test group 11, the test was carried out in the same manner as in Test group 8 except that the culture solution of *Aureobasidium* was prepared so as to have a β-glucan concentration of 0.1 g/100 g. In the case of Test group 12, the dead bacterial cells of the lactic acid bacterium ($6.7\times10^9$ cells) were suspended in PBS, and the suspension was orally administered every day during the test period by the sonde method once a day.

[Table 6]

| | | Administration form and method | | | | |
|---|---|---|---|---|---|---|
| | | For two weeks before the day of virus infection (per day) | Until day 5 of virus infection (per day) | Administration period | Dose per day | Number of mice |
| Test group 8 | | Culture solution 200 μl + lactic acid bacterium | Culture solution 200 μl + lactic acid bacterium | β-glucan 29 weeks Lactic acid bacterium 29 days | β-glucan 0.4 mg Lactic acid bacterium 6.7×10⁹ cells | 5 |
| Test group 9 | | Culture solution 200 μl + lactic acid bacterium | Culture solution 200 μl + lactic acid bacterium | β-glucan 29 weeks Lactic acid bacterium 29 days | β-glucan 0.4 4mg Lactic acid bacterium 10.1×10⁹ cells | 5 |
| Test group 10 | | Culture solution 200 μl + lactic acid bacterium | Culture solution 200 μl + lactic acid bacterium | β-glucan 29 weeks Lactic acid bacterium 29 days | β-glucan 0.4 mg Lactic acid bacterium 3.4×10⁹ cells | 5 |
| Test group 11 | | Culture solution 200 μl + lactic acid bacterium | Culture solution 200 μl + lactic acid bacterium | β-glucan 29 weeks Lactic acid bacterium 29 days | β-glucan 0.2 mg Lactic acid bacterium 6.7×10⁹ cells | 5 |
| Test group 12 | | PBS 200 μl + lactic acid bacterium | PBS 200 μl + lactic acid bacterium | Lactic acid bacterium 29 days | Lactic acid bacterium 6.7×10⁹ cells | 4 |
| Control group 3 | | PBS 200 μl | PBS 200 μl | - | - | 5 |

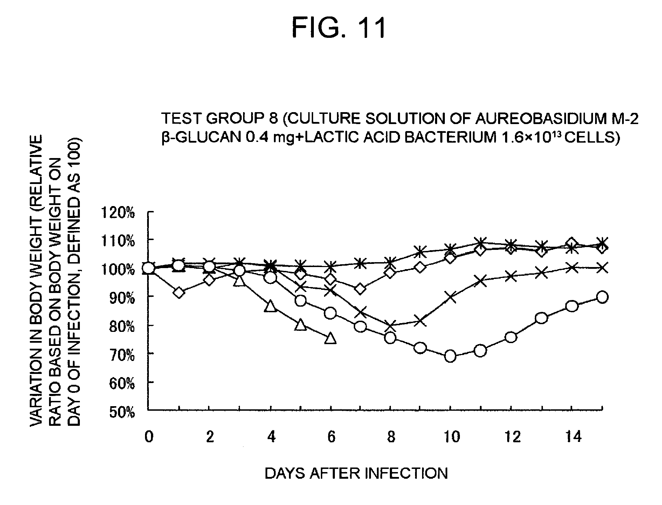

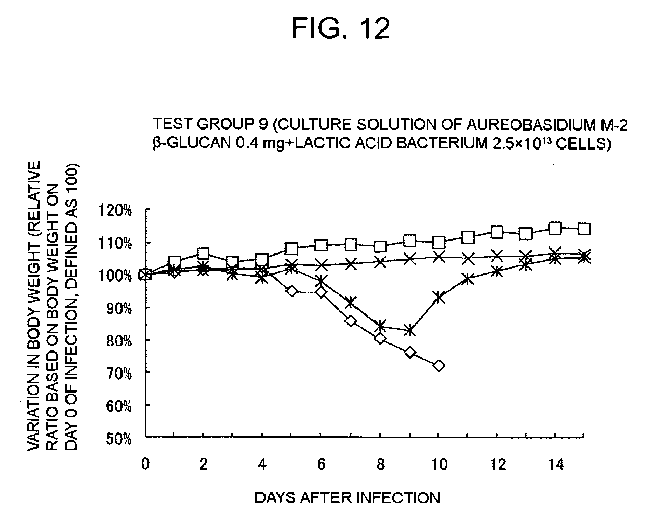

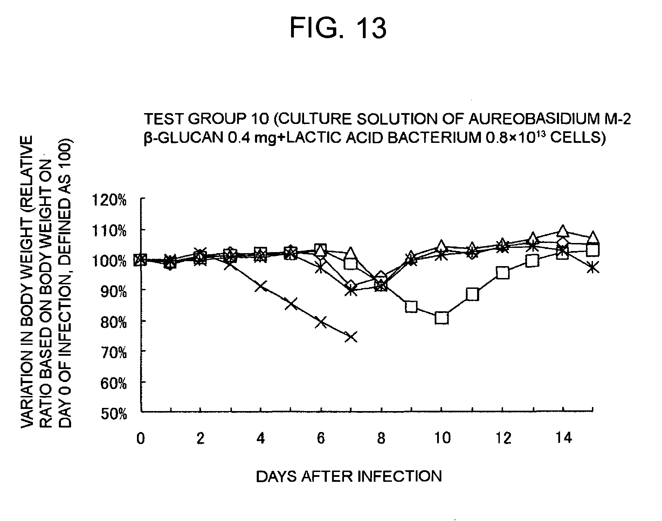

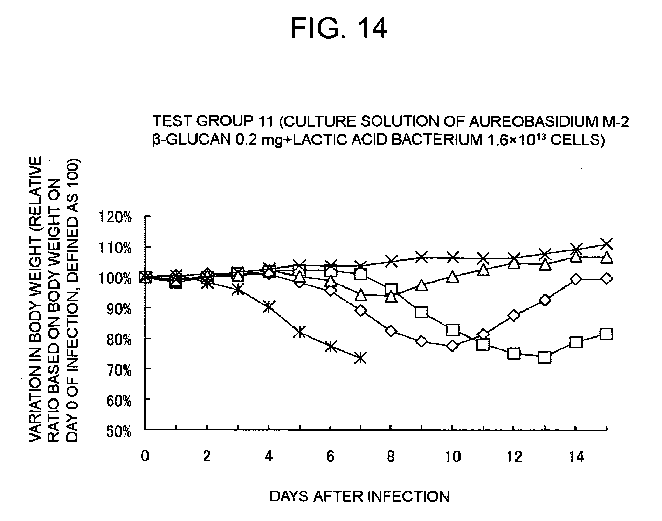

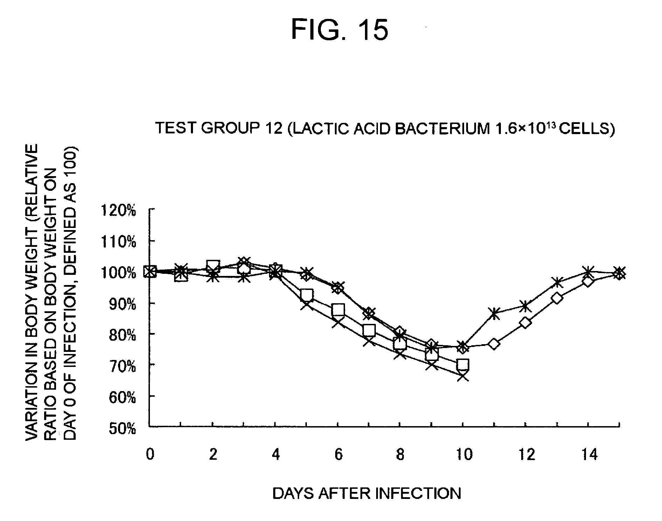

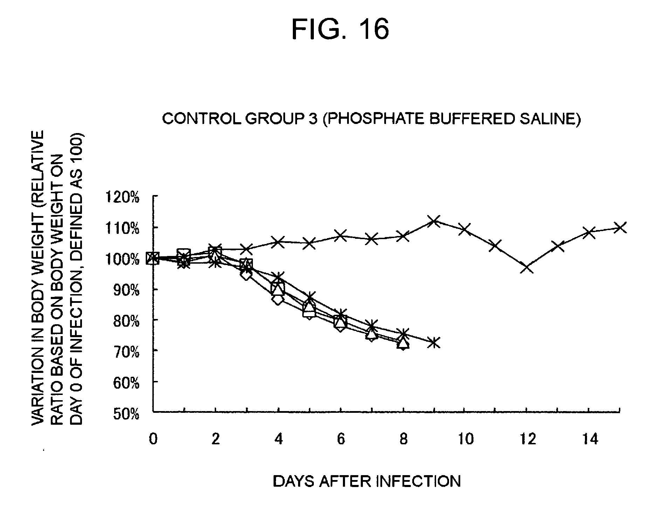

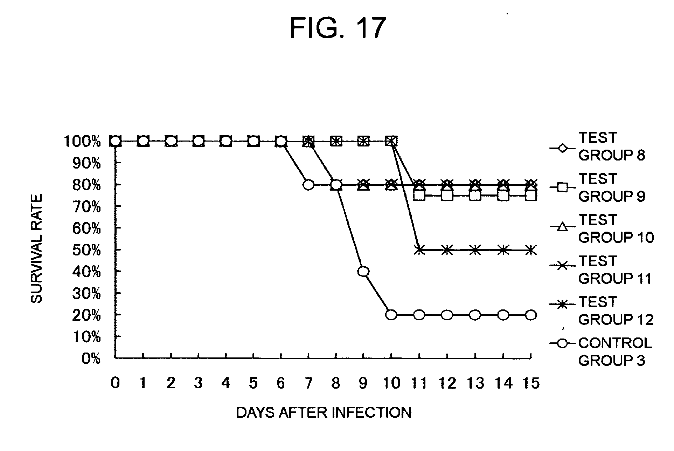

FIG. 11 illustrates the results of Test group 8, FIG. 12 illustrates the results of Test group 9, FIG. 13 illustrates the results of Test group 10, FIG. 14 illustrates the results of Test group 11, FIG. 15 illustrates the results of Test group 12, and FIG. 16 illustrates the results of Control group 3. Further, FIG. 17 illustrates transition of survival rates of mice of the respective groups.

In Test Example 3, as in the case of Test Example 2, as a result of infection of the mice carried out so that the virus titer was twice as large as that in Test Example 1, in the case of Control group 3, to which PBS was administered (FIG. 16), four of five mice died until day 9 of infection (FIG. 17).

Further, in the case of Test group 12, to which the heat-sterilized bacterial cell powder of the lactic acid bacterium was administered (FIG. 15), the body weights of four of four mice decreased by up to about 33.3%, and two of the four mice (50%) died during the test period (FIG. 17).

On the other hand, as illustrated in FIGS. 11 to 14, in the cases of Test groups 8 to 11, to each of which the culture solution of *Aureobasidium* was administered in combination with the heat-sterilized bacterial cell powder of the lactic acid bacterium, of a total of 19 mice in the test groups, only one mouse died in each group during the test period. In addition, each group included at least two mice which showed no body weight loss, and the total number of the mice was nine. The body weights of the other mice recovered to near normal levels in the test period. In particular, in the cases of Test groups 9 and 10, the body weights of the mice excluding one mouse which died in each test group decreased by up to about 19.16% and then significantly recovered to normal levels.

The above-mentioned results show that the β-glucan-containing composition derived from *Aureobasidium* is effective for treating an influenza virus infectious disease, in particular, for preventing the disease from becoming serious and promoting the healing of the disease after virus infection, and the effect can be more enhanced by using bacterial cells of a lactic acid bacterium in combination.

(Test Example 4) (Virus titer in lung of mouse)

In order to study mechanisms of the effects shown in Test Examples 1 to 3, influences on a virus titer in the lung of a mouse infected with the influenza virus were examined. Specifically, test solutions were prepared for the test groups and control group shown in Table 7 below, respectively, and orally administered, and the virus titer in the lung of each mouse infected with the influenza virus was examined. It should be noted that the phrase "dead bacterial cells of EF lactic acid bacterium" in the table means a commercially available heat-sterilized bacterial cell powder of *Enterococcus faecalis.*

[Table 7]

|  | Test solution | Form | Concentration of $\beta$-glucan |
|---|---|---|---|
| Test group 13 | Culture solution of *Aureobasidium* (M-2) | Culture solution diluted with PBS | 0.2 g/100 g |
| Test group 14 | Culture solution of *Aureobasidium* (M-2) + dead bacterial cells of EF lactic acid bacterium $6.7 \times 10^9$ cells | Suspension of dead bacterial cells of EF lactic acid bacterium in culture solution | 0.2 g/100 g |
| Test group 15 | 0.5 mg/1 mL oseltamivir | PBS solution | - |
| Control group 4 | Phosphate buffered saline (PBS) | - | - |

The test solutions were administered in the forms and by the methods shown in Table 8 below. That is, the mice were infected with the influenza virus in the same manner as in Test Example 2 except that the period after the beginning of administration of the test solutions was changed to one week. In the case of Test group 13, the test solution was prepared so that the culture solution of *Aureobasidium pullulans* M-2 (FERM BP-10014) had a $\beta$-glucan concentration of 0.2 g/100 g, and orally administered once a day in an amount of 200 $\mu$L every day during the test period by the sonde method. In the case of Test group 14, the test was carried out in the same manner as in Test group 13 except that the dead bacterial cells of the lactic acid bacterium ($6.7 \times 10^9$ cells) were used in combination as a suspension in 200 $\mu$L of the culture solution of *Aureobasidium.* In addition, in the case of Test group 15, to which only oseltamivir was administered, in order to carry out comparison with the maximum effect of administration of only oseltamivir, oseltamivir was orally administered continuously every day (morning and evening, in an amount of 200 $\mu$L per dose) after virus infection.

The virus titer in the lung of a mouse was measured as follows.

On day 1, day 3, and day 5 of virus infection, three mice of each group were dissected, and the lung washed with PBS to remove blood was collected. The collected lung was crushed, and the tissue was separated as a cell suspension and then centrifuged at 5, 000 rpm for 10 minutes. The supernatant was collected and used as a sample. The collected sample was diluted serially in 10-fold steps with 1$\times$MEM to concentrations ranging from $10^{-1}$ to $10^{-6}$. On the other hand, MDCK cells were cultured from the previous day, and a state in which the MDCK cells (one sample: one 12-well plate) were proliferated to confluency was confirmed. Then, the medium of the MDCK cells was removed, and the cells were washed with 1 ml of PBS. PBS used for washing was removed, and each of the samples diluted serially was added in an amount of 100 $\mu$L, and then the plate was allowed to stand still in a 5% $CO_2$ incubator (37°C) for one hour. During incubation, the plate was shaken every 15 minutes to prevent the cells from drying. Subsequently, 2$\times$MEM was mixed with an equal amount of a 1.6% agar solution, and trypsin was added so as to reach a final concentration of 0.0005% to prepare a MEM-agar solution. The solution was warmed in a water bath at 42°C. One hour later, the virus solution was removed, and the cells were washed with 1 ml of PBS. The prepared MEM-agar solution was added to each well in an amount of 1 ml, and the plate was allowed to stand for a while until the agar was solidified. After that, the cells were cultured in a 5% $CO_2$ incubator (35°C), and 48 hours later, the number of plaques was counted

[Table 8]

| | | Administration form and method | | | | |
|---|---|---|---|---|---|---|
| | | For one week before the day of virus infection (per day) | Until day 1, day 3, and day 5 of virus infection (per day) | Administration period | Dose per day | Number of mice |
| Test group 13 | | Culture solution 200 $\mu$l | Culture solution 200 $\mu$l | 8, 10, and 12 days | $\beta$-glucan 0.4 mg | 3/per administration period |
| Test group 14 | | Culture solution 200 $\mu$l + lactic acid bacterium | Culture solution 200 $\mu$l + lactic acid bacterium | 8, 10, and 12 days | $\beta$-glucan 0.4 mg Lactic acid bacterium $6.7 \times 10^9$ cells | 3/per administration period |
| Test group 15 | | PBS 200 $\mu$l | Oseltamivir 200 $\mu$l (twice in morning and evening, 400 $\mu$l in total) | 1, 3, and 5 days | Oseltamivir 200 $\mu$l | 3/per administration period |
| Control group 4 | | PBS 200 $\mu$l | PBS 200 $\mu$l | - | - | 3/per administration period |

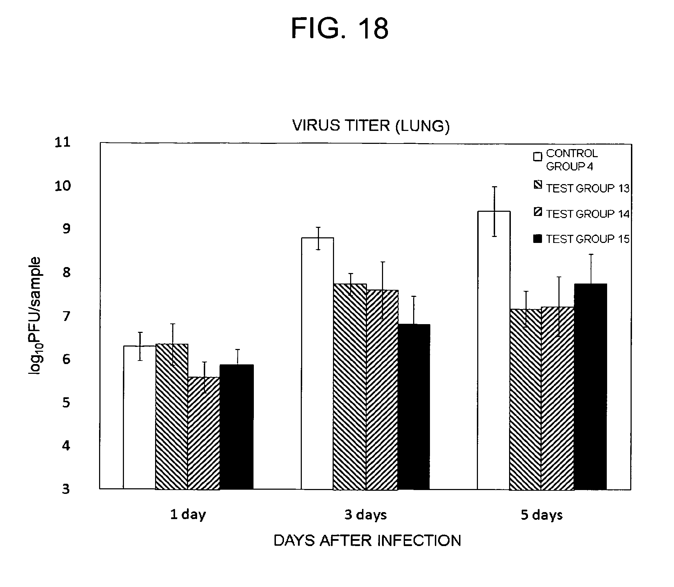

FIG. 18 illustrates the results.

The results show that on day 1 of infection, Control group 4, to which PBS was administered, had a mouse lung virus titer of 6.31 ($\log_{10}$PFU), Test group 13, to which the culture solution of *Aureobasidium* was administered, had a mouse lung virus titer of 6.36 ($\log_{10}$PFU), Test group 14, to which the culture solution of *Aureobasidium* was administered in combination with the lactic acid bacterium had a mouse lung virus titer of 5.59 ($\log_{10}$PFU), and Test group 15, to which oseltamivir (active ingredient in an anti-influenza drug Tamiflu) used as a positive control was administered, had a mouse lung virus titer of 5.88 ($\log_{10}$PFU). On day 3 and day 5 of infection, the virus titers of Control group 4, to which PBS was administered, were 8.80 ($\log_{10}$PFU) and 9.43 ($\log_{10}$PFU), respectively, and increased 154-fold and 2,057-fold, respectively, as compared to day 1 of infection. Further, on day 3 and day 5 of infection, the virus titers of Test group 13, to which the culture solution of *Aureobasidium* was administered, were 7.75 ($\log_{10}$PFU) and 7.18 ($\log_{10}$PFU), respectively, and increased 22.8-fold and 8.3-fold, respectively, as compared to day 1 of infection. In addition, the virus titers of Test group 14, to which the culture solution of *Aureobasidium* was administered in combination with the lactic acid bacterium were 7.61 ($\log_{10}$PFU) and 7.24 ($\log_{10}$PFU), respectively, and increased 81.4-fold and 40.1-fold, respectively, as compared to day 1 of infection, and increases in the virus titers of Test group 14 were suppressed as compared to Control group 4. On the other hand, when only oseltamivir, used as an active ingredient of the anti-influenza drug Tamiflu, was administered (Test group 15), on day 3 and day 5 of infection, the virus titers were 6.83 ($\log_{10}$PFU) and 7.76 ($\log_{10}$PFU), respectively, and increased 15.4-fold and 122-fold, respectively, as compared to day 1 of infection.

The results are collectively shown in Table 9. That is, the virus titer of Control group 4, to which PBS was administered, on each day after infection is defined as 1, and the virus titers of the test groups on each corresponding day after infection are tabulated.

[Table 9]

| | Comparison of virus titers (virus titer for administration of PBS on each day after infection is defined as 1) | | |
|---|---|---|---|
| | 1 day | 3 days | 5 days |
| Control group 4 | 1 | 1 | 1 |
| Test group 13 | 1/2 | 1/12 | 1/425 |
| Test group 14 | 1/9 | 1/16 | 1/442 |
| Test group 15 | 1/5 | 1/53 | 1/89 |

As shown in Table 9, when the virus titer of Control group 4, to which PBS was administered, was defined as 1, the virus titers of Test group 13, to which the culture solution of *Aureobasidium* was administered, were 1/12 on day 3 and 1/425 on day 5, and the virus titers of Test group 14, to which the culture solution of *Aureobasidium* was administered in combination with the lactic acid bacterium, were 1/16 on day 3 and 1/442 on day 5. Therefore, administration of the culture solution of *Aureobasidium* (Test group 13) or administration of the culture solution of *Aureobasidium* in combination with the lactic acid bacterium (Test group 14) was considered to effectively suppress proliferation of the virus in the lung. It should be noted that as shown in the results of Test group 14, in this test example, when the culture solution of *Aureobasidium* was administered in combination with the bacterial cells of the lactic acid bacterium on day 1 of infection, an increase in the virus titer was further suppressed as compared to administration of only the culture solution of *Aureobasidium.*

(Test Example 5) (Mouse lung T cell activity and NK cell activity)

In order to study mechanisms of the effects shown in Test Examples 1 to 3, activated T cells and activated NK cells in the lung of each mouse infected with the influenza virus were examined. Specifically, test solutions were prepared for the test groups and control group shown in Table 10 below, respectively, and orally administered, and the numbers of T cells and activated T cells among all the T cells and the numbers of NK cells and activated NK cells among all the NK cells in the lung of each mouse infected with the influenza virus were examined on day 5. It should be noted that the phrase "dead bacterial cells of EF lactic acid bacterium" in the table means a commercially available heat-sterilized bacterial cell powder of *Enterococcus faecalis.*

[Table 10]

| | Test solution | Form | Concentration of β-glucan |
|---|---|---|---|
| Test group 16 | Culture solution of *Aureobasidium* (M-2) | Culture solution diluted with PBS | 0.2 g/100 g |
| Test group 17 | Culture solution of *Aureobasidium* (M-2) + dead bacterial cells of EF lactic acid bacterium $6.7 \times 10^9$ cells | Suspension of dead bacterial cells of EF lactic acid bacterium in culture solution | 0.2 g/100 g |
| Test group 18 | 0.5 mg/1 mL oseltamivir | PBS solution | - |
| Control group 5 | Phosphate buffered saline (PBS) | - | - |

The test solutions were administered in the same manner as in Test Example 4 above in the forms and by the methods shown in Table 11 below. That is, the mice were infected with the influenza virus in the same manner as in Test Example 2 except that the period after the beginning of administration of the test solutions was changed to one week. In the case of Test group 16, the test solution was prepared so that the culture solution of *Aureobasidium pullulans* M-2 (FERM BP-10014) had a β-glucan concentration of 0.2 g/100 g, and orally administered once a day in an amount of 200 μL every day during the test period by the sonde method. In the case of Test group 17, the test was carried out in the same manner as in Test group 16 except that the dead bacterial cells of the lactic acid bacterium ($6.7 \times 10^9$ cells) were used in combination as a suspension in 200 μL of the culture solution of *Aureobasidium*. In addition, in the case of Test group 17, to which only oseltamivir was administered, in order to carry out comparison with the maximum effect of administration of only oseltamivir, oseltamivir was orally administered every day (morning and evening, in an amount of 200 μL per dose) continuously after virus infection.

The numbers of the activated T cells and activated NK cells in the lung of each mouse were counted as follows. That is, three mice of each group were dissected on day 5 of virus infection, and the lung washed with PBS to remove blood was collected. 4.9 ml of HEPES buffer, 100 μl of collagenase D (100 mg/Ml in HEPES), and 10 μl of DNase 1 were added to the lung of one mouse, and the mixture was homogenized to crush the tissue in part. The homogenate was shaken at 37°C for 30 minutes and treated enzymatically. The mixture was homogenized again to crush the tissue completely. The crushed lung tissues of three mice in each group were collected and passed through a mesh to remove extra components other than cells, and the cells were washed with PBS repeatedly, followed by counting the number of the cells.

The cell suspension was dispensed in 96 wells in an amount of $2 \times 10^6$ cells/well per sample, and "LEAF Purified anti-Mouse CD16/32" was added in an amount of 1 μl/well (in 100 μl FACS buffer) for blocking to prevent extra cells from adhering to antibodies. The plate was allowed to stand still on ice for 30 minutes. CD3 APC antibody against T cells, NK 1.1 FITC antibody against NK cells, PE Cy7-CD69 antibody against activated T cells, and PE Cy7-CD69 antibody against activated NK cells were added thereto in an amount of 1 µl/well, and the plate was allowed to stand still at 4°C for 30 minutes while the plate was shielded from light. The plate was washed with FACS buffer several times to remove antibodies which were not bound to the cells, and 7-AAD for labeling and distinguishing dead cells was added in an amount of 10 µl per sample. An FACS analysis was carried out using a flow cytometer/cell sorter apparatus, and the ratio of the NK cells or T cells in viable cells was determined. In addition, the ratio of activated cells in the respective cells was determined.

[Table 11]

| | Administration form and method | | | | |
|---|---|---|---|---|---|
| | For one week before the day of virus infection (per day) | Until day 1, day 3, and day 5 of virus infection (per day) | Administration period | Dose per day | Number of mice |
| Test group 16 | Culture solution 200 µl | Culture solution 200 µl | 12 days | β-glucan 0.4 mg | 3/per administration period |
| Test group 17 | Culture solution 200 µl + lactic acid bacterium | Culture solution 200 µl + lactic acid bacterium | 12 days | β-glucan 0.4 mg Lactic acid bacterium 6.7×10⁹ cells | 3/per administration period |
| Test group 18 | PBS 200 µl | Oseltamivir 200 µl (twice in morning and evening, 400 µl in total) | 5 days | Oseltamivir 0.2 mg | 3/per administration period |
| Control group 5 | PBS 200 µl | PBS 200 µl | - | - | 3/per administration period |

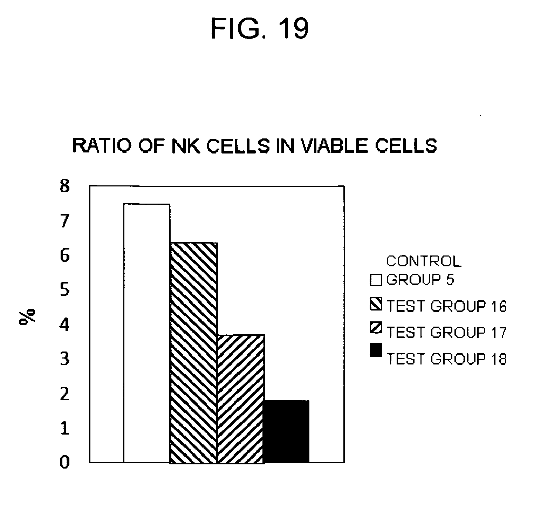

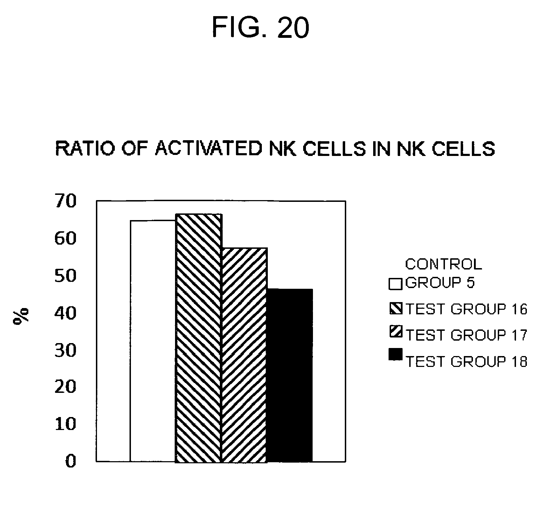

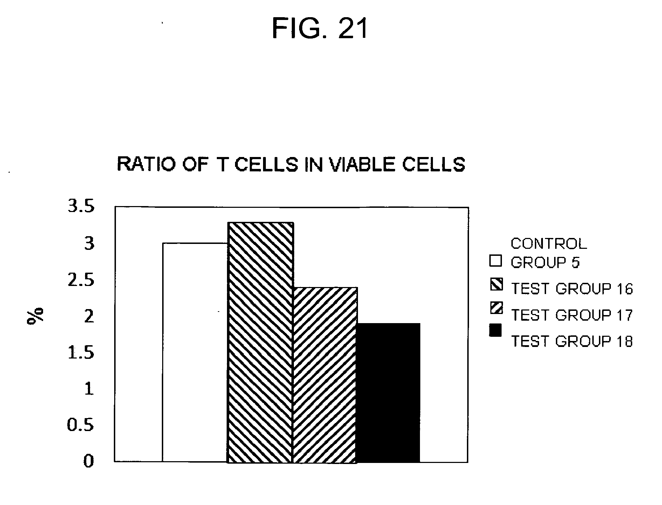

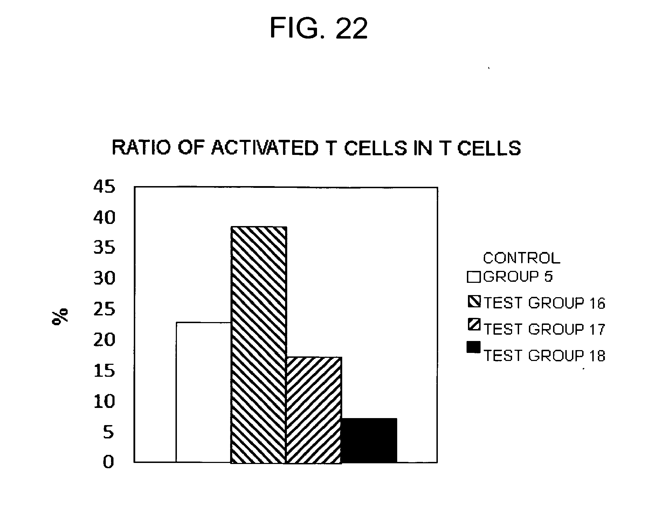

FIGS. 19 to 22 illustrate the results.
On day 5 of infection, with regard to NK cells which play an important role in natural immunity, the ratios of the NK cells in viable cells in the lung of Control group 5, to which PBS was administered, Test group 16, to which the culture solution of *Aureobasidium* was administered, and Test group 17, to which the culture solution of *Aureobasidium* was administered in combination with the lactic acid bacterium, were found to be 7.5%, 6.4%, and 3.7%, respectively. The ratio in the case of Test group 18, to which oseltamivir (active ingredient in the anti-influenza drug Tamiflu) was administered, was found to be about 1.8%, which was only about one-quarter of that of Control group (the results are illustrated in FIG. 19). Meanwhile, the ratios of activated NK cells in the NK cells of Control group 5, to which PBS was administered, Test group 16, to which the culture solution of *Aureobasidium* was administered, and Test group 17, to which the culture solution of *Aureobasidium* was administered in combination with the lactic acid bacterium, were found to be 64.7%, 66.4%, and 57.2, respectively. On the other hand, the ratio in the case of Test group 18, to which oseltamivir (active ingredient in the anti-influenza drug Tamiflu) was administered, was found to be only 46.2% (the results are illustrated in FIG. 20). Activation of the NK cells is given as one reason for the fact that proliferation of the influenza virus in the cases of Test groups 16 and 17 was suppressed. As shown in Test Example 4 (FIG. 18) above, in the cases of Test group 16 (corresponding to Test group 13 in Test Example 4 above) and Test group 17 (corresponding to Test group 14 in Test Example 4 above), proliferation of the influenza virus was suppressed to 1/425 in Test group 13 and 1/442 in Test group 14 as compared to Control group 5, to which PBS was administered, which suggests that there is a mechanism to suppress the influenza virus by a route other than enhancement of NK cell activation. On the other hand, the number of the activated NK cells of Test group 18 was smaller than those of the other groups. As one reason for this, it was estimated that oseltamivir acted directly on the influenza virus as an NA inhibitor for the influenza virus to suppress proliferation and was not involved in activation of the NK cells.
Next, with regard to T cells, which play an important role in acquired immunity, the number of T cells in lung viable cells of each group on day 5 of virus infection was very small (about 3%), and there were few differences among the numbers of the cells (the results are illustrated in FIG. 21). However, the ratios of activated T cells in the T cells of Control group 5, to which PBS was administered, and Test group 16, to which the culture solution of *Aureobasidium* was administered, were found to be 22.9% and 38.5%, respectively, and the cells were activated 1.68 times as compared to Control group 5. In addition, the ratio of activated T cells of Test group 17, to which the culture solution of *Aureobasidium* was administered in combination with the lactic acid bacterium was found to be 17.3%. Further, the ratio of activated T cells of Test group 18, to which oseltamivir (active ingredient in the anti-influenza drug Tamiflu) was administered, was found to be 7.3%, and the number of the activated T cells was only about one-third of that of Control group 5, to which PBS was administered (the results are illustrated in FIG. 22).

As mentioned above, it was suggested that administration of only the culture of *Aureobasidium* or administration of the culture of *Aureobasidium* in combination with the lactic acid bacterium increased the number of activated T cells or NK cells as compared to administration of oseltamivir (active ingredient in the anti-influenza drug Tamiflu) and not only suppressed invasion and proliferation of the influenza virus but also prevented symptoms caused by infection with the influenza virus from becoming serious. Further, it was suggested that administration of the culture of *Aureobasidium* or the culture of *Aureobasidium* in combination with the lactic acid bacterium possibly achieved smooth transition from natural immunity to acquired immunity, and as a result, contributed to reduction in the weight loss ratio or death rate.

It should be noted that it is considered that the culture of *Aureobasidium* and oseltamivir (active ingredient in the anti-influenza drug Tamiflu) have different mechanisms for protection from infection with the influenza virus, and hence as shown in Test group 6 in Test Example 2 above (FIG. 10), it was suggested that administration of the culture of *Aureobasidium* in combination with oseltamivir can suppressed invasion and proliferation of the influenza virus but also prevented symptoms caused by infection with the influenza virus from becoming serious more effectively.

## Claims

1. A therapeutic agent comprising, as an active ingredient, a β-glucan-containing composition obtainable from a culture of a microorganism belonging to *Aureobasidium* sp., for therapeutic use in a method for preventing an influenza virus infectious disease from becoming serious and for promoting healing of the influenza virus infectious disease.

2. The therapeutic agent according to claim 1 for use according to claim 1, wherein the microorganism belonging to *Aureobasidium* sp. is *Aureobasidium pullulans* M-1 having an accession number of FERM BP-08615 or *Aureobasidium pullulans* M-2 having an accession number of FERM BP-10014.

3. The therapeutic agent according to claim 1 or 2 for use according to claim 1 or 2, respectively, further comprising, as another active ingredient, bacterial cells of a lactic acid bacterium.

4. The therapeutic agent according to claim 3 for use according to claim 3, wherein the bacterial cells of a lactic acid bacterium are dead bacterial cells of *Enterococcus faecalis.*

5. The therapeutic agent according to any one of claims 1 to 4 for use according to any one of claims 1 to 4, respectively, which is for use by oral administration.

6. The therapeutic agent according to any one of claims 1 to 5 for use according to any one of claims 1 to 5, respectively, which suppresses proliferation of the influenza virus in the lung.

7. The therapeutic agent according to any one of claim 1 to 6 for use according to any one of claims 1 to 6, respectively, which is for use by administration in combination with at least one kind selected from the group consisting of an influenza neuraminidase inhibitor, an antiviral drug, an antibiotic, and a steroid drug.

8. Use of a β-glucan-containing composition obtainable from a culture of a microorganism belonging to *Aureobasidium* sp., in the manufacture of a therapeutic agent for preventing an influenza virus infectious disease from becoming serious and for promoting healing of the influenza virus infectious disease.

9. Use according to claim 8, wherein the microorganism belonging to *Aureobasidium* sp. is *Aureobasidium pullulans* M-1 having an accession number of FERM BP-08615 or *Aureobasidium pullulans* M-2 having an accession number of FERM BP-10014.

10. Use according to claim 8 or 9, wherein the therapeutic agent further comprises, as another active ingredient, bacterial cells of a lactic acid bacterium.

11. Use according to claim 10, wherein the bacterial cells of a lactic acid bacterium are dead bacterial cells of *Enterococcus faecalis.*

**12.** Use according to any one of claims 8 to 11, wherein the therapeutic agent is for use by oral administration.

**13.** Use according to any one of claims 8 to 12, wherein the therapeutic agent suppresses proliferation of the influenza virus in the lung.

**14.** Use according to any one of claims 8 to 13, wherein the therapeutic agent is for administration in combination with at least one kind selected from the group consisting of an influenza neuraminidase inhibitor, an antiviral drug, an antibiotic, and a steroid drug.

**Patentansprüche**

**1.** Therapeutisches Mittel, das als aktiven Bestandteil eine β-Glucan enthaltende Zusammensetzung umfasst, die aus einer Kultur eines zu *Aureobasidium* sp. gehörenden Mikroorganismus erhältlich ist, zur therapeutischen Verwendung in einem Verfahren zur Vermeidung, dass eine Influenzavireninfektionserkrankung bedenklich wird, und zur Förderung der Heilung der Influenzavireninfektionserkrankung.

**2.** Therapeutisches Mittel nach Anspruch 1 zur Verwendung nach Anspruch 1, worin der zu *Aureobasidium* sp. gehörende Mikroorganismus *Aureobasidium pullulans* M-1 mit der Zugangsnummer FERM BP-08615 oder *Aureobasidium pullulans* M-2 mit der Zugangsnummer FERM BP-10014 ist.

**3.** Therapeutisches Mittel nach Anspruch 1 oder 2 zur Verwendung nach Anspruch 1 bzw. 2, das des Weiteren Bakterienzellen eines Milchsäurebakteriums als weiteren aktiven Bestandteil umfasst.

**4.** Therapeutisches Mittel nach Anspruch 3 zur Verwendung nach Anspruch 3, worin die Bakterienzellen eines Milchsäurebakteriums tote Bakterienzellen von *Enterococcus faecalis* sind.

**5.** Therapeutisches Mittel nach einem der Ansprüche 1 bis 4 zur jeweiligen Verwendung nach einem der Ansprüche 1 bis 4, das der Verwendung durch orale Verabreichung dient.

**6.** Therapeutisches Mittel nach einem der Ansprüche 1 bis 5 zur jeweiligen Verwendung nach einem der Ansprüche 1 bis 5, das die Proliferation des Influenzavirus in der Lunge unterdrückt.

**7.** Therapeutisches Mittel nach einem der Ansprüche 1 bis 6 zur jeweiligen Verwendung nach einem der Ansprüche 1 bis 6, das der Verwendung durch Verabreichung in Kombination mit zumindest einer aus der aus einem Influenzaneuramidinidaseinhibitor, einem antiviralen Arzneimittel, einem Antibiotikum und einem Steroidarzneimittel bestehenden Gruppe ausgewählten Art umfasst.

**8.** Verwendung einer β-Glucan enthaltenden Zusammensetzung, die aus einer Kultur eines zu *Aureobasidium* sp. gehörenden Mikroorganismus erhältlich ist, zur Herstellung eines therapeutischen Mittels zur Vermeidung, dass eine Influenzavireninfektionserkrankung bedenklich wird, und zur Förderung der Heilung der Influenzavireninfektionserkrankung.

**9.** Verwendung nach Anspruch 8, worin der zu *Aureobasidium* sp. gehörende Mikroorganismus *Aureobasidium pullulans* M-1 mit der Zugangsnummer FERM BP-08615 oder *Aureobasidium pullulans* M-2 mit der Zugangsnummer FERM BP-10014 ist.

**10.** Verwendung nach Anspruch 8 oder 9, worin das therapeutische Mittel des Weiteren Bakterienzellen eines Milchsäurebakteriums als weiteren aktiven Bestandteil umfasst.

**11.** Verwendung nach Anspruch 10, worin die Bakterienzellen eines Milchsäurebakteriums tote Bakterienzellen von *Enterococcus faecalis* sind.

**12.** Verwendung nach einem der Ansprüche 8 bis 11, worin das therapeutische Mittel der Verwendung durch orale Verabreichung dient.

**13.** Verwendung nach Anspruch 8 bis 12, worin das therapeutische Mittel die Proliferation des Influenzavirus in der Lunge unterdrückt.

**14.** Verwendung nach einem der Ansprüche 8 bis 13, worin das therapeutische Mittel der Verabreichung in Kombination mit zumindest einer aus der aus einem Influenzaneuramidinidaseinhibitor, einem antiviralen Arzneimittel, einem Antibiotikum und einem Steroidarzneimittel bestehenden Gruppe ausgewählten Art umfasst.

**Revendications**

**1.** Agent thérapeutique comprenant, comme ingrédient actif, une composition contenant du ß-glucane pouvant être obtenu d'une culture d'un microorganisme appartenant à *Aureobasidium* sp., pour l'utilisation thérapeutique dans une méthode pour empêcher qu'une maladie infectieuse provoquée par le virus de la grippe devienne sérieuse et pour promouvoir la guérison de la maladie infectieuse provoquée par le virus de la grippe.

**2.** Agent thérapeutique selon la revendication 1 pour utilisation selon la revendication 1, dans laquelle le microorganisme appartenant à *Aureobasidium* sp. est le *Aureobasidium pullulans* M-1 ayant un nombre d'accès de FERM BP-08615 ou le *Aureobasidium pullulans* M-2 ayant un nombre d'accès de FERM BP-10014.

**3.** Agent thérapeutique selon la revendication 1 ou 2 pour utilisation selon la revendication 1 ou 2, respectivement, comprenant en outre, comme autre ingrédient actif, des cellules bactériennes d'une bactérie d'acide lactique.

**4.** Agent thérapeutique selon la revendication 3 pour utilisation selon la revendication 3, où les cellules bactériennes d'une bactérie d'acide lactique sont des cellules bactériennes mortes de *Enterococcus faecalis.*

**5.** Agent thérapeutique selon l'une quelconque des revendications 1 à 4 pour utilisation selon l'une quelconque des revendications 1 à 4, respectivement, qui est pour l'utilisation par administration orale.

**6.** Agent thérapeutique selon l'une quelconque des revendications 1 à 5 pour utilisation selon l'une quelconque des revendications 1 à 5, respectivement, qui supprime la prolifération du virus de la grippe dans le poumon.

**7.** Agent thérapeutique selon l'une quelconque des revendications 1 à 6 pour utilisation selon l'une quelconque des revendications 1 à 6 respectivement, qui est pour l'utilisation par administration en combinaison avec au moins un type sélectionné dans le groupe consistant en un inhibiteur de neuraminidase de la grippe, un médicament antiviral, un antibiotique et un médicament stéroîde.

**8.** Utilisation d'une composition contenant du ß-glucane pouvant être obtenue d'une culture d'un microorganisme appartenant à *Aureobasidium* sp., dans la fabrication d'un agent thérapeutique pour empêcher qu'une maladie infectieuse provoquée par le virus de la grippe devienne sérieuse et pour promouvoir la guérison de la maladie infectieuse provoquée par le virus de la grippe.

**9.** Utilisation selon la revendication 8, où le microorganisme appartenant à *Aureobasidium* sp. est *Aureobasidium pullulans* M-1 ayant un numéro d'accès de FERM BP-08615 ou *Aureobasidium pullulans* M-2 ayant un numéro d'accès de FERM BP-10014.

**10.** Utilisation selon la revendication 8 ou 9, où l'agent thérapeutique comprend en outre, comme autre ingrédient actif, des cellules bactériennes d'une bactérie d'acide lactique.

**11.** Utilisation selon la revendication 10, où les cellules bactériennes d'une bactérie d'acide lactique sont des cellules bactériennes mortes *d'Enterococcus faecalis.*

**12.** Utilisation selon l'une quelconque des revendications 8 à 11, où l'agent thérapeutique est pour l'utilisation par administration orale.

**13.** Utilisation selon l'une quelconque des revendications 8 à 12, où l'agent thérapeutique supprime la prolifération du virus de la grippe dans le poumon.

**14.** Utilisation selon l'une quelconque des revendications 8 à 13, où l'agent thérapeutique est pour l'administration en combinaison avec au moins un type sélectionné dans le groupe consistant en inhibiteur de neuraminidase de la grippe, un médicament antiviral, un antibiotique et un médicament stéroïde.

# FIG. 1

TEST GROUP 1 (CULTURE SOLUTION OF AUREOBASIDIUM M-1)

VARIATION IN BODY WEIGHT (RELATIVE RATIO BASED ON BODY WEIGHT ON DAY 0 OF INFECTION, DEFINED AS 100)

94.2%

84.7%

DAYS AFTER INFECTION

# FIG. 2

TEST GROUP 2 (OSELTAMIVIR)

80.4%

74.8%

VARIATION IN BODY WEIGHT (RELATIVE RATIO BASED ON BODY WEIGHT ON DAY 0 OF INFECTION, DEFINED AS 100)

DAYS AFTER INFECTION

# FIG. 3

CONTROL GROUP 1 (PHOSPHATE BUFFERED SALINE)

# FIG. 4

TEST GROUP 3 (CULTURE SOLUTION OF AUREOBASIDIUM M-1)

Y-axis: VARIATION IN BODY WEIGHT (RELATIVE RATIO BASED ON BODY WEIGHT ON DAY 0 OF INFECTION, DEFINED AS 100)

X-axis: DAYS AFTER INFECTION

# FIG. 5

TEST GROUP 4 (CULTURE SOLUTION OF AUREOBASIDIUM M-2)

VARIATION IN BODY WEIGHT (RELATIVE RATIO BASED ON BODY WEIGHT ON DAY 0 OF INFECTION, DEFINED AS 100)

DAYS AFTER INFECTION

# FIG. 6

TEST GROUP 5 (CULTURE SOLUTION OF AUREOBASIDIUM M-1+LACTIC ACID BACTERIUM)

VARIATION IN BODY WEIGHT (RELATIVE RATIO BASED ON BODY WEIGHT ON DAY 0 OF INFECTION, DEFINED AS 100)

DAYS AFTER INFECTION

FIG. 7

TEST GROUP 6 (CULTURE SOLUTION OF AUREOBASIDIUM M-1+ OSELTAMIVIR)

# FIG. 8

TEST GROUP 7 (OSELTAMIVIR)

# FIG. 9

CONTROL GROUP 2 (PHOSPHATE BUFFERED SALINE)

VARIATION IN BODY WEIGHT (RELATIVE RATIO BASED ON BODY WEIGHT ON DAY 0 OF INFECTION, DEFINED AS 100)

DAYS AFTER INFECTION

# FIG. 10

# FIG. 11

TEST GROUP 8 (CULTURE SOLUTION OF AUREOBASIDIUM M-2 β-GLUCAN 0.4 mg+LACTIC ACID BACTERIUM $1.6 \times 10^{13}$ CELLS)

# FIG. 12

TEST GROUP 9 (CULTURE SOLUTION OF AUREOBASIDIUM M-2 β-GLUCAN 0.4 mg+LACTIC ACID BACTERIUM 2.5×10$^{13}$ CELLS)

# FIG. 13

TEST GROUP 10 (CULTURE SOLUTION OF AUREOBASIDIUM M-2 β-GLUCAN 0.4 mg+LACTIC ACID BACTERIUM $0.8 \times 10^{13}$ CELLS)

Y-axis: VARIATION IN BODY WEIGHT (RELATIVE RATIO BASED ON BODY WEIGHT ON DAY 0 OF INFECTION, DEFINED AS 100)

X-axis: DAYS AFTER INFECTION

# FIG. 14

TEST GROUP 11 (CULTURE SOLUTION OF AUREOBASIDIUM M-2
β-GLUCAN 0.2 mg+LACTIC ACID BACTERIUM $1.6 \times 10^{13}$ CELLS)

# FIG. 15

TEST GROUP 12 (LACTIC ACID BACTERIUM 1.6×10$^{13}$ CELLS)

# FIG. 16

CONTROL GROUP 3 (PHOSPHATE BUFFERED SALINE)

# FIG. 17

# FIG. 18

# FIG. 19

## RATIO OF NK CELLS IN VIABLE CELLS

CONTROL
□ GROUP 5
☒ TEST GROUP 16
☑ TEST GROUP 17
■ TEST GROUP 18

# FIG. 20

**RATIO OF ACTIVATED NK CELLS IN NK CELLS**

CONTROL
☐ GROUP 5
◣ TEST GROUP 16
▨ TEST GROUP 17
■ TEST GROUP 18

# FIG. 21

## RATIO OF T CELLS IN VIABLE CELLS

Legend:
- □ CONTROL GROUP 5
- ▨ TEST GROUP 16
- ▧ TEST GROUP 17
- ■ TEST GROUP 18

# FIG. 22

RATIO OF ACTIVATED T CELLS IN T CELLS

CONTROL
□ GROUP 5
◨ TEST GROUP 16
◪ TEST GROUP 17
■ TEST GROUP 18

**EP 2 486 931 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005220065 A

- JP 57149301 A

**Non-patent literature cited in the description**

- **DIRK B. MENDEL et al.** *ANTIMICROBIAL AGENTS AND CHEMOTHERAPY,* March 1998, 640-646